# EUROPEAN PATENT SPECIFICATION

(11) **EP 2 701 680 B1**
(45) Date of publication and mention of the grant of the patent: **31.10.2018**
(21) Application number: 12718867.0
(22) Date of filing: 27.04.2012
(51) Int. Cl.: A61F 9/00, A61K 9/70, A61K 47/34, A61K 31/5575, A61K 31/216, A61K 9/00

(54) **SUSTAINED RELEASE LATANOPROST IMPLANT**
LATANOPROST-IMPLANTAT MIT VERZÖGERTER FREISETZUNG
IMPLANT À BASE DE LATANOPROST À LIBÉRATION SOUTENUE

(30) Priority: 29.04.2011 US 201161480657 P; 29.04.2011 US 201161480630 P
(43) Date of publication of application: 05.03.2014
(73) Proprietor: ALLERGAN, INC., Irvine, CA 92612 (US)
(72) Inventor: GHEBREMESKEL, Alazar, N., Irvine, California 92606 (US); SPADA, Lon, T., Walnut, California 91789 (US)
(74) Representative: Hoffmann Eitle
(86) International application number: PCT/US2012/035398
(87) International publication number: WO 2012/149278

(56) References cited:
- EP-A2- 1 944 032
- WO-A2-2009/035562
- US-A1- 2007 260 203
- US-A1- 2010 104 654
- MERKLI A ET AL: "USE OF INSOLUBLE BIODEGRADABLE POLYMERS IN OPHTHALMIC SYSTEMS FOR THE SUSTAINED RELEASE OF DRUGS", EUROPEAN JOURNAL OF PHARMACEUTICS AND BIOPHARMACEUTICS, ELSEVIER SCIENCE PUBLISHERS B.V., AMSTERDAM, NL, vol. 41, no. 5, 1 October 1995 (1995-10-01), pages 271-283, XP000535194, ISSN: 0939-6411

## Description

### BACKGROUND OF THE INVENTION

### 1. Field of the Invention.

The present invention relates to biodegradable intraocular implants that provide for the sustained release of latanoprost for reducing intraocular pressure in an eye of an individual in need thereof, and to methods of making these implants.

### 2. Summary of the Related Art

Latanoprost is a prostaglandin F_{2α} analogue which is indicated in the treatment of open-angle glaucoma or ocular hypertension in patients who are intolerant of other intraocular pressure (IOP)-lowering medications or insufficiently responsive (i.e., failed to achieve target IOP after multiple measurements over time) to another IOP-lowering medication. Latanoprost may be used alone or in combination with other antiglaucoma agents.

Latanoprost is an isopropyl ester prodrug of an analog of prostaglandin F_{2α}. It is hydrolyzed by esterases in the cornea to latanoprost acid, which is biologically active. The elimination of latanoprost acid from plasma is rapid (half-life 17 minutes) after either ophthalmic or intravenous administration. Latanoprost's pharmacology makes it a candidate for formulation as an IOP lowering sustained release polymer implant. However, its physicochemical properties make it an extremely challenging molecule to incorporate into a biodegradable implant. That is, Latanoprost (MW 432.58) is a colorless to slightly yellow oil that is very soluble in acetonitrile and freely soluble in acetone, ethanol, ethyl acetate, isopropanol, methanol and octanol. It is practically insoluble in water.

It is believed that a latanoprost sustained release implant would be an effective treatment for long-term reduction of intraocular pressure associated with glaucoma or other ocular diseases.

US2010104654 (A1) discloses biocompatible, bioerodible implants and microspheres including latanoprost and a biodegradable polymer which are effective to treat glaucoma when placed in an intraocular location (such as into the subtenon space).

EP1944032 (A2) discloses microspheres comprising a prostamide component and a biodegradable polymer matrix. This disclosure also relates to biodegradable intraocular implants.

US2007260203 (A1) discloses intraocular implants comprising a vasoactive compound and a polymer useful for treating an ocular condition. According to this disclosure, the vasoactive compound can be a vasodilator, the polymer can be a biodegradable polymer, and the ocular condition can be glaucoma.

### Brief Summary of the Invention

The present invention provides a sustained release biodegradable polymer implant containing latanoprost for reducing intraocular pressure in an eye of an individual in need thereof as defined in claim 1. In one embodiment of the present disclosure, latanoprost is incorporated into a solid biodegradable implant, wherein said implant comprises a mixture of a polylactide (PLA) and a lactide-glycolide copolymer (PLGA). The implant in such an embodiment of the present disclosure is fabricated by combining latanoprost, PLA and PLGA polymers by melt extrusion or by dissolving the components in a solvent and removing the solvent to recover a solid composite, e.g. a polymeric film or fiber, which may be fabricated into a sustained release polymer implant containing latanoprost. The composite, in the form of a thin film, can be cut to any shape and dimension. For example, a disc shaped implant that is about 100 µM to about 500 µM thick and about 2 to about 6 mm in diameter may be formed from a thin film comprising latanoprost. In ophthalmic use, this thin implant may be rolled-up and inserted into the subTenon's space through a small opening. Once inserted, it may unfurl either partially or completely to its original disc shape providing a large surface for drug diffusion through the sclera.

Accordingly, one embodiment is an intraocular implant comprising or consisting of latanoprost and a biodegradable polymer matrix for reducing intraocular pressure in an eye of an individual in need thereof. The latanoprost can be incorporated in the polymer matrix. In certain embodiments the latanoprost is homogenously distributed throughout the polymer matrix.

The implant is prepared in the form of an extruded filament (i.e, fiber). The extruded filament can be prepared by a hot-melt extrusion method, as described herein.

The biodegradable polymer matrix in the extruded filament (i.e., fiber) comprises or consists of a poly(D,L-lactide), or a combination of a poly(D,L-lactide) with one or more of a poly(D-lactide), a poly(L-lactide), a polyglycolide, or a poly(D,L-lactide-co-glycolide) (PLGA).

The percent of D,L-lactide in the PLGA can be about 0 to less than about 100%, about 15-85%, or about 35-65%. In specific embodiments the molar ratio of D,L-lactide to glycolide in the PLGA copolymer is about 75:25 or about 50:50.

The filament (or fiber) is extruded and can be made by a hot-melt extrusion method and may comprise about 20-30% (w/w) latanoprost, about 40-80% (w/w) of a poly(D,L-lactide-co-glycolide), and about 0-20% of a poly(D,L-lactide), with the proviso that the extruded filament comprises an acid terminated poly(D,L-lactide) having an inherent viscosity of about 0.16-0.24 dl/g (**R202H**).

In another embodiment, the filament comprises about 20-30% w/w latanoprost, 40-60% (w/w) of a poly(D,L-lactide-co-glycolide), 0-20% of a poly(D,L-lactide), and 0-10% w/w PEG 3350, with the proviso that the extruded filament comprises an acid terminated poly(D,L-lactide) having an inherent viscosity of about 0.16-0.24 dl/g (**R202H**).

In another embodiment, the fiber comprises a mixture of poly(D,L-lactide) (PLA) and poly(D,L-lactide-co-glycolide) (PLGA) polymers. In a more specific embodiment, the fiber comprises from about 20 to about 60 percent w/w PLA and from about 10 to about 50 percent w/w PLGA.

RESOMER® R202H is an acid terminated poly(D,L-lactide) having an inherent viscosity of about 0.16-0.24 dl/g, as measured for a 0.1% solution in chloroform at 25°C.

RESOMER® R208 is an ester terminated poly(D,L-lactide) having an inherent viscosity of about 1.8-2.2 dl/g, as measured for a 0.1% solution in chloroform at 25°C.

RESOMER® R202S is an ester terminated poly(D,L-lactide) having an inherent viscosity of about 0.16-0.24 dl/g, as measured for a 0.1% solution in chloroform at 25°C. RESOMER® R203S is an ester terminated poly(D,L-lactide) having an inherent viscosity of about 0.25-0.35 dl/g, as measured for a 0.1% solution in chloroform at 25°C.

RESOMER® RG752S is an ester terminated poly(D,L-lactide-co-glycolide) having an inherent viscosity of about 0.16-0.24 dl/g (as measured for a 0.1% solution in chloroform at 25°C), and a D,L-lactide:glycolide ratio of about 75:25.

RESOMER® RG755S is an ester terminated poly(D,L-lactide-co-glycolide) having an inherent viscosity of about 0.50-0.70 dl/g (as measured for a 0.1% solution in chloroform at 25°C), and a D,L-lactide:glycolide ratio of about 75:25.

In another embodiment, the intraocular implant is in the form of an extruded fiber comprising latanoprost incorporated in a biodegradable polymer matrix.

The extruded intraocular implant (i.e., fiber) may further comprise a polyethylene glycol (PEG). The PEG can act as a plasticizing agent, making it possible to extrude the implant (i.e, to form the fiber) at temperatures lower than might otherwise be possible in the absence of the PEG. Lower extrusion temperatures are desirable to preserve latanoprost activity.

In one embodiment the PEG plasticizer is PEG 3350.

One embodiment provides for a biodegradable intraocular implant for reducing intraocular pressure in an eye of an individual in need thereof, the implant comprising latanoprost and a biodegradable polymer, wherein:
a) the implant does not contain bimatoprost;
b) the implant is in the form of an extruded filament;
c) said filament optionally further comprises a polyethylene glycol (PEG);
d) said biodegradable polymer is an acid terminated poly(D,L-lactide) having an inherent viscosity of about 0.16-0.24 dl/g (**R202H**), or a combination of **R202H** and one or more of i), ii), iii) iv), or v):
   i) an ester terminated poly(D,L-lactide) having an inherent viscosity of about 1.8-2.2 dl/g (**R208**);
   ii) an ester terminated poly(D,L-lactide) having an inherent viscosity of about 0.16-0.24 dl/g (**R202S**);
   iii) an ester terminated poly(D,L-lactide) having an inherent viscosity of about 0.25-0.35 dl/g (**R203S**);
   iv) an ester terminated poly(D,L-lactide-co-glycolide) having an inherent viscosity of about 0.16-0.24 dl/g and a D,L-lactide to glycolide ratio of about 75:25 (**RG752S**); or
   v) an ester terminated poly(D,L-lactide-co-glycolide) having an inherent viscosity of about 0.50-0.70 dl/g and a D,L-lactide to glycolide ratio of about 75:25 (**RG755S**);
e) wherein the solubility parameters for each of the latanoprost, the biodegradable polymer(s), and the PEG differ one from the other by no more than 10 MPa^{1/2}; and wherein
f) the implant releases latanoprost continuously for at least 30 days after placement in the eye of the individual.

In another embodiment, the implant does not contain a prostamide.

The filament can comprise about 5 to about 10% by weight PEG 3350.

In one embodiment the extruded latanoprost-containing filament does not contain polyethylene glycol.

In one embodiment the extruded filament comprises or consists of about 20% by weight latanoprost, about 40% by weight of an ester terminated poly(D,L-lactide) having an inherent viscosity of about 0.16-0.24 dl/g (**RG202S**), and about 40% by weight of an acid terminated poly(D,L-lactide) having an inherent viscosity of about 0.16-0.24 dl/g (**R202H**).

The invention provides a biodegradable intraocular implant for use in a method of reducing intraocular pressure (i.e., treating ocular hypertension) in a subject in need, the method comprising placing an ocular implant into an eye of the subject, the implant comprising latanoprost and a biodegradable polymer. The ocular implant is an extruded filament as described herein. The implant can be placed into the vitreous body, subconjunctival space, subTenon's space, or anterior chamber of the eye, for example.

Another embodiment provides for a method of making a biodegradable intraocular implant, the method comprising: mixing PEG 3350, the latanoprost, and the biodegradable polymer(s), to form a mixture; and extruding the mixture at a temperature of less than about 80°C to form a filament that will release latanoprost continuously for at least 30 days after placement in the eye of the individual.

### Definitions

"About" means that the number, range, value or parameter so qualified encompasses ten percent more and ten percent less of the number, range, value or parameter.

A "patient in need" can be a human or non-human mammal suffering from an ocular condition. The ocular condition may be a condition treatable with a latanoprost-containing implant. Examples of an ocular condition treatable with a latanoprost-containing implant include glaucoma and elevated intraocular pressure (or ocular hypertension).

"Treat", "treating", or "treatment" means a reduction or resolution or prevention of an ocular condition. A treatment is usually effective to reduce at least one symptom of an ocular condition.

"Therapeutically effective amount" means the level or amount of agent needed to treat an ocular condition, or reduce a symptom associated with the condition without causing significant negative or adverse side effects to the eye or a region of the eye. In view of the above, a therapeutically effective amount of latanoprost is an amount that is effective in reducing intraocular pressure in an eye of an individual.

"Therapeutic component" means that portion of an implant other than the polymer matrix comprising one therapeutic agent used to treat an ocular condition. The therapeutic component can be a discrete region of an implant, or it may be homogenously distributed throughout the implant.

"Biodegradable polymer" means a polymer or polymers which degrade in vivo, and wherein erosion of the polymer or polymers over time occurs concurrent with or subsequent to release of the therapeutic agent, e.g, the latanoprost. The terms "biodegradable" and "bioerodible" are equivalent and are used interchangeably herein. A biodegradable polymer may be a homopolymer, a copolymer, or a polymer comprising more than two different polymeric units. The polymer can be a gel or hydrogel type polymer. Examples include PLA or PLGA polymers or mixtures or derivatives thereof.

Prostamides (prostaglandin-ethanolamides) have been described by, for example, Woodward et al. (2007) British Journal of Pharmacology 150:342-352. Prostamides have been disclosed in, for example, U.S. Pat. Nos. 6,395,787 and 6,403,649.

The solubility parameter for a substance is a numerical value which indicates the relative solvency behavior of that substance. The concept is discussed and defined in US 2008/0145403.

### Brief Description of the Drawings

Figures 1A-C show release profiles for latanoprost-containing formulations of Example 1.
Figures 2A-C show release profiles for latanoprost-containing formulations of Example 2.

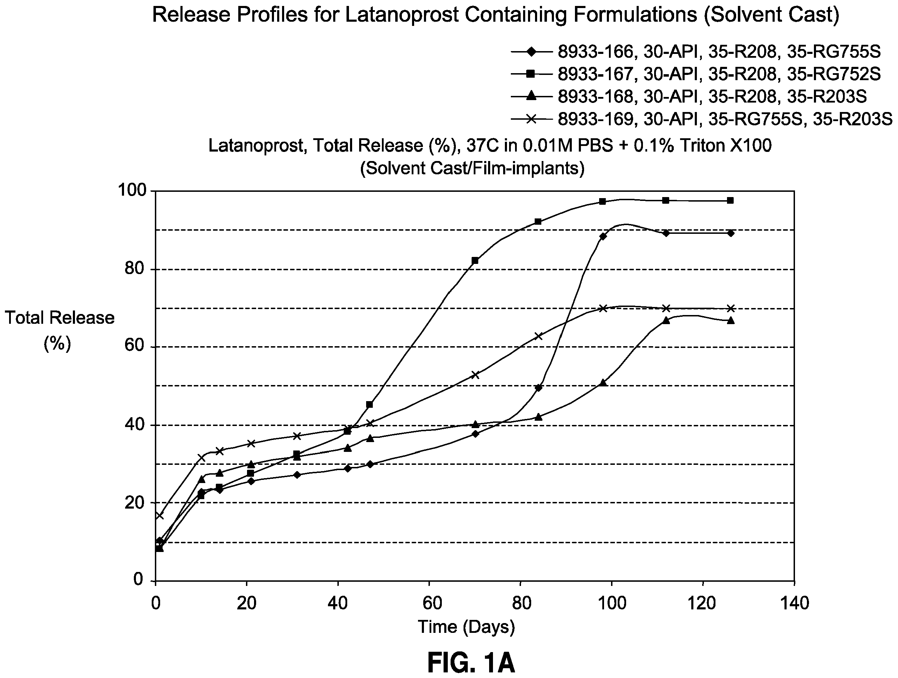

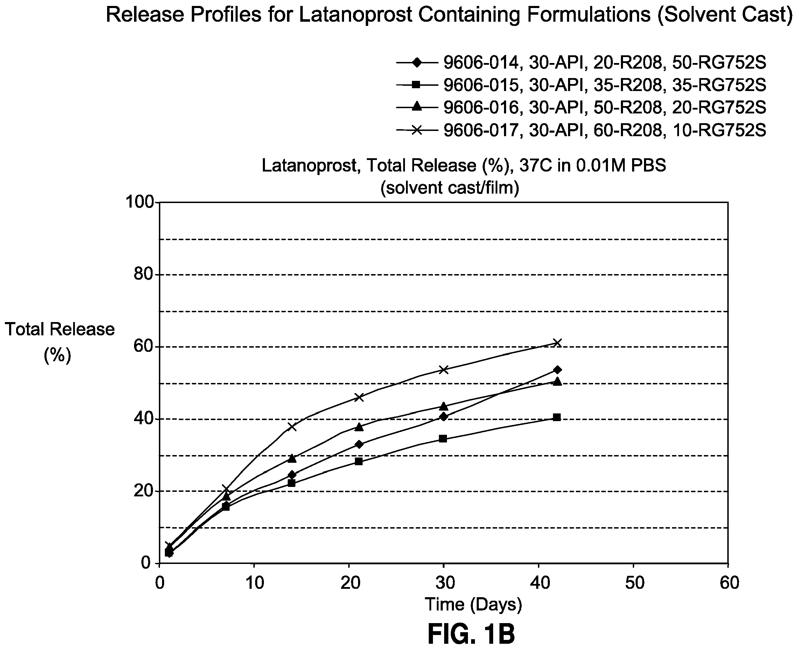

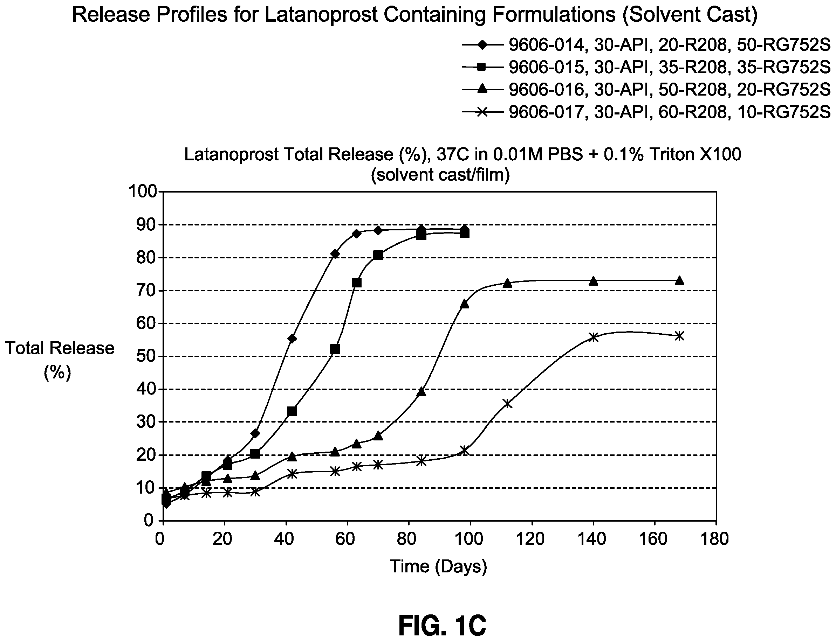

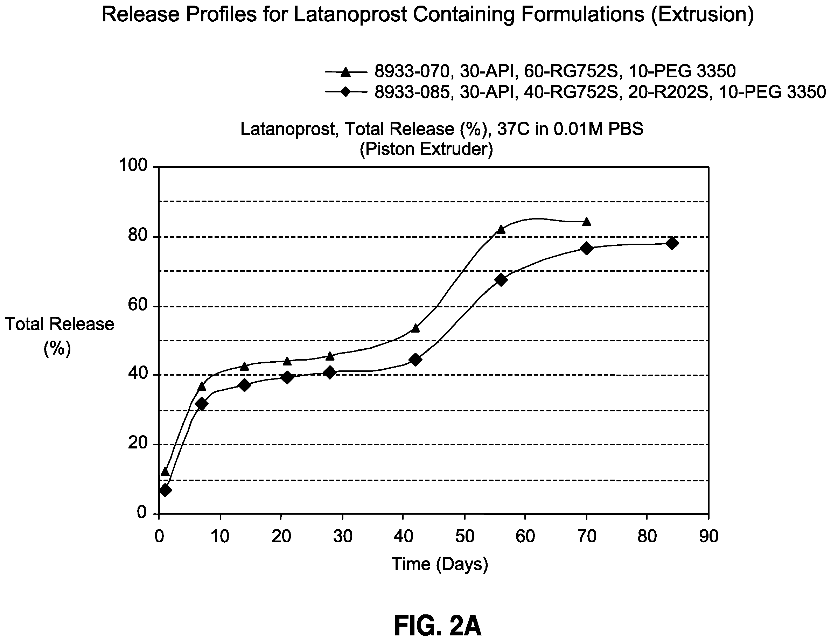

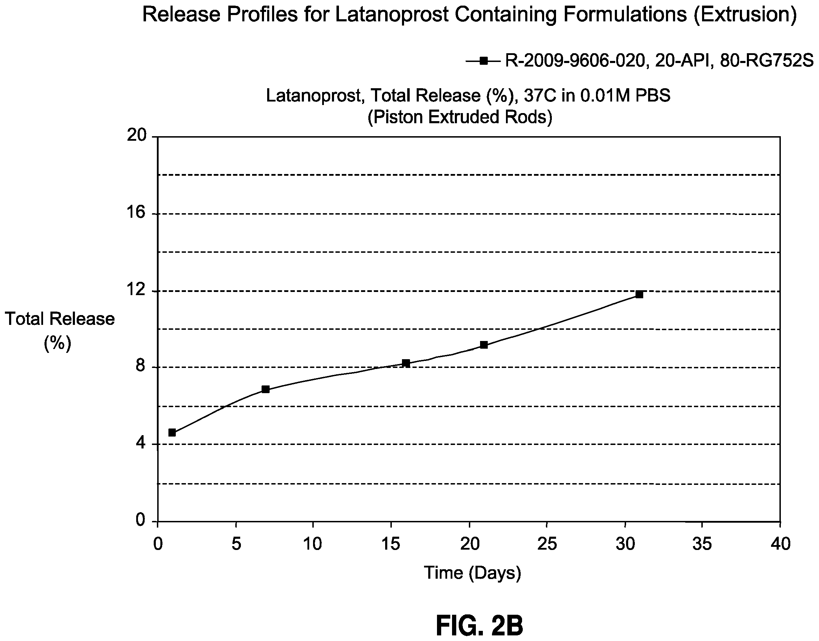

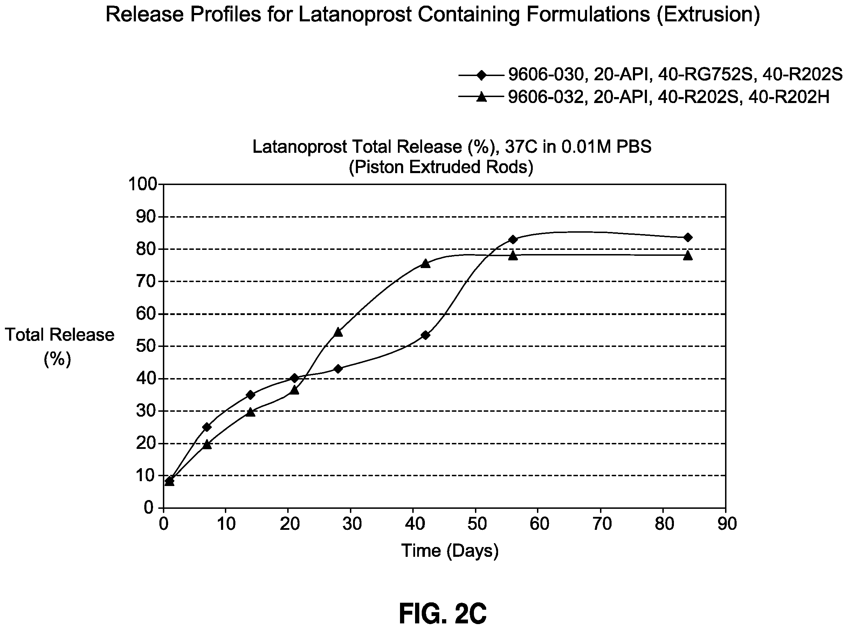

The implants in Figures 1A-C and Figures 2A and 2B do not form part of the invention and are provided for information purposes only. The implant in Figure 2C containing RG752S similarly does not form part of the invention and is provided for information purposes only.

### Detailed Description of the Invention

Latanoprost is very difficult to incorporate into biodegradable polymer matrices, because it is an oil. It has now been discovered, that this problem can be overcome by using high molecular weight PLA or a combination of high molecular weight PLA and PLGA polymers and fabricating the implant by melt extrusion. The oily drug substance (DS), i.e. latanoprost, helps plasticize the polymer and allows the production of a homogeneous polymer.

For purposes of forming an extruded filament that will provide for a long-lasting extended and perhaps nearly linear rate of latanoprost release, the solubility parameters of the latanoprost, biodegradable polymer(s), co-solvent (e.g., PEG 3350) preferably differ by no more than about 10 MPa^{0.5} from one another. In one embodiment, the solubility parameters of the components chosen for inclusion in the extruded implant or film differ by no more than about 7 MPa^{0.5}, one from the other.

The solubility parameter of PEG 3350 is about 20 MPa^{0.5}.

When preparing an extruded filament with latanoprost, it is important to keep the extrusion temperature as low as possible to avoid loss and degradation of the latanoprost. The temperatures ordinarily needed to extrude drug-containing biodegradable polymer implants can cause the latanoprost to degrade and/or bubble out of the mixture, thereby causing undesirable loss of the oily drug. This can be overcome by using a select combination of low molecular weight polymers and a plasticizer such as a polyethylene glycol (PEG) that are compatible with the drug substance. The oily DS (e.g. latanoprost) and PEG plasticize the polymers to a degree that allows the mixture to be extruded at a temperature where the DS is not degraded or lost. In some embodiments, the latanoprost-containing polymer mixture is extruded at a temperature of about 50°C, from about 50°C to about 55°C, less than about 60°C, less than about 70°C, less than about 80°C, or at a temperature from about 50°C to less than about 80°C.

An extruded filament may be cut to implant lengths of from about 5 µm to about 10 mm, e.g. about 1, 2, or 3 mm, or from about 10 µm to about 1 mm. The implants may have any appropriate length so long as the diameter of the implant permits the implant to move through a needle. The filament can have a diameter of less than about 500 µm, or from about 500 µm to about 1.5 mm.

The total weight of an implant can be less than about 250 µg or from about 250-5000 µg or more. For example, an extruded implant may weigh about 500 µg, about 1000 µg, or about 2000 µg.

Latanoprost (CAS Registry No. 130209-82-4) is disclosed in US Patent Numbers 6,429,226; 6,417,230; 6,187,813; 6,030,999; 5,849,791; 5,627,208; 5,578,618; 5,422,368 and 5,296,504 and has the following structure:

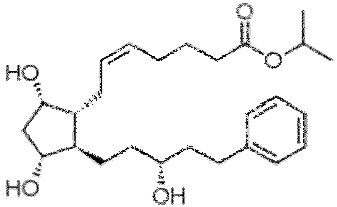

In the present invention, latanoprost is incorporated into a biodegradable polymer composite by melt extrusion. The solubility parameter for latanoprost is about 22 to about 24 MPa^{0.5}.

Suitable polymeric materials or compositions for use in the implant include those materials, which are compatible, i.e. biocompatible, with the eye so as to cause no substantial interference with the functioning or physiology of the eye. Such materials preferably are at least partially and more preferably substantially completely biodegradable or bioerodible or bioabsorbable.

The biodegradable polymer of the invention comprises acid terminated poly(D,L-lactide) having an inherent viscosity of about 0.16-0.24 dl/g (**R202H**). Other polymers of hydroxyaliphatic carboxylic acids, either homopolymers or copolymers, may be used in conjunction with R202H. Polyesters of interest include polymers of D-lactic acid, L-lactic acid, racemic lactic acid and glycolic acid, and combinations thereof. Generally, by employing the L-lactate or D-lactate, a slowly eroding polymer or polymeric material is achieved, while erosion is substantially enhanced with the lactate racemate.

Some preferred characteristics of the polymers or polymeric materials for use in the present invention may include biocompatibility, compatibility, ease of use of the polymer in making the implant of the present invention, a half-life in the physiological environment of at least about 6 hours, preferably greater than about one day, and not significantly increasing the viscosity of the vitreous.

The biodegradable polymeric materials, which are included to form the implant, are desirably subject to enzymatic or hydrolytic instability. Water-soluble polymers may be cross-linked with hydrolytic or biodegradable unstable cross-links to provide useful water insoluble polymers. The degree of stability can be varied widely, depending upon the choice of monomer, whether a homopolymer or copolymer is employed, employing mixtures of polymers, and whether the polymer includes terminal acid groups.

Equally important to controlling the biodegradation of the polymer is the relative average molecular weight of the polymeric composition employed in the implant. Different molecular weights of the same or different polymeric compositions may be included in the implant to modulate the release profile. In certain implants, the relative average molecular weight of the polymer will range from about 9 to about 200 kD, usually from about 10 to about 54 kD, and more usually from about 12 to about 45 kD.

In some implants, copolymers of glycolic acid and lactic acid are used together with acid terminated poly(D,L-lactide) having an inherent viscosity of about 0.16-0.24 dl/g (**R202H**), where the rate of biodegradation is controlled by the ratio of glycolic acid to lactic acid. The most rapidly degraded copolymer has roughly equal amounts of glycolic acid and lactic acid. Homopolymers, or copolymers having ratios other than equal, are more resistant to degradation. The ratio of glycolic acid to lactic acid will also affect the brittleness of the implant, where a more flexible implant is desirable for larger geometries. The percent of polylactic acid in the polylactic acid polyglycolic acid (PLGA) copolymer can be 0-100%, preferably about 15-85%, more preferably about 35-65%, with the proviso that the biodegradable intraocular implant comprises acid terminated poly(D,L-lactide) having an inherent viscosity of about 0.16-0.24 dl/g (**R202H**). In some implants, a 50/50 PLGA copolymer is used.

The biodegradable polymer matrix of the intraocular implant comprises a mixture of two or more biodegradable polymers, selected from the group consisting of PLA, PLGA and mixtures thereof, with the proviso that the biodegradable intraocular implant comprises acid terminated poly(D,L-lactide) having an inherent viscosity of about 0.16-0.24 dl/g (**R202H**). For example, the implant may comprise a mixture of a first biodegradable polymer and a different second biodegradable polymer, wherein at least one or more of the biodegradable polymers may have terminal acid groups.

PLA/PLGA polymers from the RESOMER® product line are available from Evonik Industries AG, Germany, and include the following:

| **Resomer** | **Monomer ratio** | **i.v. dL/g** |
|---|---|---|
| RG502 | 50:50 poly (D, L-lactide-co-glycolide) | 0.2 |
| RG502H | 50:50 poly (D, L-lactide-co-glycolide), acid end | 0.2 |
| RG503 | 50:50 poly (D, L-lactide-co-glycolide) | 0.3 |
| RG504 | 50:50 poly (D, L-lactide-co-glycolide) | 0.5 |
| RG505 | 50:50 poly (D, L-lactide-co-glycolide) | 0.7 |
| RG506 | 50:50 poly (D, L-lactide-co-glycolide) | 0.8 |
| RG752 | 75:25 poly (D,L lactide-co-glycolide) | 0.2 |
| RG755 | 75:25 poly(D,L lactide-co-glycolide) | 0.6 |
| RG756 | 75:25 poly(D,L lactide-co-glycolide) | 0.8 |
| RG858 | 85:15 poly (D,L-lactide-co-glycolide) | 1.4 |
| R202H | poly (D, L-lactide), acid end | 0.2 |
| R203 | poly (D,L-lactide) | 0.3 |
| R206 | poly (D,L-lactide) | 0.6 |
| R104 | poly (D,L-lactide) | (3500) |

Release of the drug (latanoprost) from an erodible polymer is the consequence of several mechanisms or combinations of mechanisms. Some of these mechanisms include desorption from the implant's surface, dissolution, diffusion through porous channels of the hydrated polymer and erosion. Erosion can be bulk or surface or a combination of both. The implant of the invention releases latanoprost continuously for at least 30 days after placement in the eye of the individual. In certain implants, therapeutic amounts of drug are released for no more than about 30-35 days after implantation. For example, an implant may release the drug at a rate effective to sustain a therapeutically effective amount of drug for about one month (i.e., about 30 days) after being placed in an eye. As another example, the implant may release drug at a rate effective to sustain a therapeutically effective amount of drug for more than forty days, such as for about six months.

The following examples are intended to illustrate the present invention.

Example 1 is provided for information purposes only and does not form part of the invention.

### Example 1

**Table 1. Latanoprost Containing Film Formulations**

| | w/w,% | | | | |
|---|---|---|---|---|---|
| **Formulation No** | **Latanoprost** | **Resomer R208** | **Resomer R203S** | **Resomer RG752S** | **Resomer RG755S** |
| R-2007-8933-166 | 30 | 35 | 0 | 0 | 35 |
| R-2007-8933-167 | 30 | 35 | 0 | 35 | 0 |
| R-2007-8933-168 | 30 | 35 | 35 | 0 | 0 |
| R-2007-8933-169 | 30 | 0 | 35 | 0 | 35 |
| R-2009-9606-014 | 30 | 20 | 0 | 50 | 0 |
| R-2009-9606-015 | 30 | 35 | 0 | 35 | 0 |
| R-2009-9606-016 | 30 | 50 | 0 | 20 | 0 |
| R-2009-9606-017 | 30 | 60 | 0 | 10 | 0 |

Release studies were performed in triplicates as follows. The dried film was cut using 4-mm biopsy punch (approximately 2.0-mg), and was placed into a 10-mL vial containing 0.01M phosphate buffered saline (pH 7.4) + 0.1% Triton X100. The samples were then transferred into a shaking water bath set at 37°C and 50 rpm. At various time-points, the solution was removed and analyzed by HPLC for the amount of released latanoprost. The removed solution is replaced with fresh phosphate buffered saline solution. Drug release profiles are shown in Figures 1A-C.

The film-shaped implants made by solvent casting can be cut to any shape and dimension. One example is a disc shaped implant 100 µm to 500 µm thick and 2 to 6 mm in diameter.

### Example 2

This example prepares an extruded solid polymer implant containing latanoprost. While the above discussion relates mainly to the problems of incorporating latanoprost in a film oily drug substances (DS) are also very difficult to incorporate into hot-melt extruded implants because they exude the oily DS when heated. It is important to keep the extrusion temperature as low as possible to avoid loss and degradation of the DS. This can be overcome by using a select combination of polymers and a plasticizer like PEG that are compatible with the drug substance. The oily DS and PEG plasticize the polymers to a degree that allows the mixture to be extruded at a temperature where the DS is not degraded or lost. Suitable formulations are shown in Table 2.

The polymer implants were made by hot-melt extrusion using a mechanically driven ram microextruder. The implants are rod-shaped, but they can be made into any geometric shape by changing the extrusion die.

The samples were initially mixed using a spatula in a weigh-boat for 15 minutes. The samples were then transferred into a stainless steel container containing two ¼" stainless steel balls and mixing continued using a Turbula mixer for two separate 15-min cycles. The powder blend was mixed by hand using a spatula between each cycle and after the final cycle. The blended material was then compacted into an extruder barrel using a pellet compactor; then the extruder barrel was placed into the heated well of the piston extruder and extruded using a 500-µm nozzle.

The implants made by hot-melt extrusion contain 20-30% Latanoprost, 40-60% of a biodegradable poly (D,L-lactide-co-glycolide) polymer (Resomer® RG752s), 0-40% of a biodegradable poly (D,L-lactide) polymer (Resomer® R202s), 0-40% of a biodegradable poly (D,L-lactide) acid end caped polymer (Resomer® R202H), and 0-10% PEG-3350. The successful hot-melt extruded invention formulations are summarized in Table 2.

**Table 2. Latanoprost Containing Extruded Filament Formulations**

| | w/w,% | | | | |
|---|---|---|---|---|---|
| **Formulation No** | **Latanoprost** | **Resomer RG752S** | **Resomer R202S** | **Resomer 202H** | **PEG-3350** |
| R-2008-8933-070* | 30 | 60 | 0 | 0 | 10 |
| R-2008-8933-085* | 30 | 40 | 20 | 0 | 10 |
| R-2009-9606-019* | 20 | 75 | 0 | 0 | 5 |
| R-2009-9606-020* | 20 | 80 | 0 | 0 | 0 |
| R-2009-9606-030* | 20 | 40 | 40 | 0 | 0 |
| R-2009-9606-032 | 20 | 0 | 40 | 40 | 0 |

| | | | | | |
|---|---|---|---|---|---|
| * These formulations are provided for information purposes only and do not form part of the invention. | | | | | |

Release studies were performed in triplicates as follows. The filaments were cut into one milligram implant (approximately 3-mm long), and was placed into a 10-mL vial containing 0.01M phosphate buffered saline (pH 7.4). The samples were then transferred into a shaking water bath set at 37°C and 50 rpm. At various time-points, the solution was removed and analyzed by HPLC for the amount of released latanoprost. The removed solution is replaced with fresh phosphate buffered saline solution. Drug release profiles are shown Figures 2A-C.

In general, considering the extruded implants of this invention, latanoprost may comprise from 10 to 60%, more preferably from 20 to 50 %, e.g. 30 %, of the implant and the implant further comprising a mixture of two or more biodegradable polymers, selected from the group consisting of PLA, PLGA and mixtures thereof, with the proviso that the extruded filament comprises an acid terminated poly(D,L-lactide) having an inherent viscosity of about 0.16-0.24 dl/g (**R202H**).

Unless otherwise specified the percentages of drug, polymer(s), and PEG (co-solvent) given above represent the mass of the component relative to the total mass of the composition or implant formulation, the ratio multiplied by 100 to give the weight/weight percentage of the component in the implant (i.e, the % w/w).

## Claims

1. A biodegradable intraocular implant for reducing intraocular pressure in an eye of an individual in need thereof, the implant comprising latanoprost and one or more biodegradable polymers, wherein:
a) the implant does not contain bimatoprost;
b) the implant is in the form of an extruded filament;
c) said filament optionally further comprises a polyethylene glycol (PEG);
d) the solubility parameters for each of the latanoprost, the biodegradable polymer(s), and the PEG differ one from the other by no more than 10 MPa^{1/2};
e) the implant releases latanoprost continuously for at least 30 days after placement in the eye of the individual; and wherein
f) one of the biodegradable polymer(s) is an acid terminated poly(D,L-lactide) having an inherent viscosity of about 0.16-0.24 dl/g (**R202H**).

2. A biodegradable implant according to claim 1, wherein the implant does not contain a prostamide.

3. A biodegradable implant according to claim 1, wherein said extruded filament comprises about 5 to about 10% by weight PEG 3350.

4. A biodegradable implant according to claim 1, said implant in the form of an extruded filament and consisting of about 20% by weight latanoprost, about 40% by weight of an ester terminated poly(D,L-lactide) having an inherent viscosity of about 0.16-0.24 dl/g (**RG202S**), and about 40% by weight of an acid terminated poly(D,L-lactide) having an inherent viscosity of about 0.16-0.24 dl/g (**R202H**).

5. A method for making a biodegradable intraocular implant according to claim 1, the method comprising:
a) mixing the latanoprost, the biodegradable polymer(s), and optionally the PEG to form a mixture; and
b) extruding the mixture at a temperature of less than about 80°C to form a filament that will release latanoprost continuously for at least 30 days after placement in the eye of the individual.

## Patentansprüche

1. Biologisch abbaubares intraokulares Implantat zum Reduzieren von intraokularem Druck in einem Auge eines Individuums, das dieses benötigt, wobei das Implantat Latanoprost und ein oder mehrere biologisch abbaubare Polymere umfasst, worin:
a) das Implantat kein Bimatoprost enthält;
b) das Implantat in Form eines extrudierten Filaments vorliegt;
c) das Filament gegebenenfalls ferner ein Polyethylenglykol (PEG) umfasst;
d) die Löslichkeitsparameter für Latanoprost, das biologisch abbaubare Polymer (die biologisch abbaubaren Polymere) und das PEG sich jeweils um nicht mehr als 10 MPa^{1/2} unterscheiden;
e) das Implantat Latanoprost kontinuierlich für mindestens 30 Tage nach der Platzierung im Auge des Individuums freisetzt; und worin
f) eines der biologisch abbaubaren Polymere ein säureterminiertes Poly(D,L-lactid) mit einer inhärenten Viskosität von etwa 0,16-0,24 dl/g (R202H) ist.

2. Biologisch abbaubares Implantat gemäß Anspruch 1, worin das Implantat kein Prostamid enthält.

3. Biologisch abbaubares Implantat gemäß Anspruch 1, worin das extrudierte Filament etwa 5 bis etwa 10 Gew.-% PEG 3350 umfasst.

4. Biologisch abbaubares Implantat gemäß Anspruch 1, worin das Implantat in Form eines extrudierten Filaments ist und aus etwa 20 Gew.-% Latanoprost, etwa 40 Gew.-% eines Poly(D,L-lactid)s mit endständigen Estergruppen und einer logarithmischen Viskositätszahl von etwa 0,16-0,24 dl/g (RG202S) und etwa 40 Gew.-% eines säureterminierten Poly(D,L-lactid) mit einer inhärenten Viskosität von etwa 0,16-0,24 dl/g (R202H) besteht.

5. Verfahren zur Herstellung eines biologisch abbaubaren intraokularen Implantats gemäß Anspruch 1, das Verfahren umfassend:
(a) Mischen des Latanoprosts, des biologisch abbaubaren Polymers (der biologisch abbaubaren Polymere) und gegebenenfalls des PEGs, so dass eine Mischung gebildet wird; und
(b) Extrudieren der Mischung bei einer Temperatur von weniger als etwa 80°C, so dass ein Filament gebildet wird, welches Latanoprost kontinuierlich für mindestens 30 Tage nach dem Einbringen in das Auge des Individuums freisetzt.

## Revendications

1. Implant intraoculaire biodégradable pour réduire la pression intraoculaire dans un oeil d'un individu en ayant besoin, l'implant comprenant du latanoprost et un ou plusieurs polymère(s) biodégradable(s), dans lequel :
a) l'implant ne contient pas de bimatoprost ;
b) l'implant se présente sous la forme d'un filament extrudé ;
c) ledit filament comprend en outre optionnellement un polyéthylèneglycol (PEG);
d) les paramètres de solubilité pour chacun du latanoprost, du(des) polymère(s) biodégradable(s) et du PEG diffèrent les uns des autres d'au plus 10 MPa^{1/2} ;
e) l'implant libère du latanoprost en continu pendant au moins 30 jours après une mise en place dans l'oeil de l'individu ; et dans lequel
f) un du(des) polymère(s) biodégradable(s) est un poly(D,L-lactide) à terminaison acide ayant une viscosité inhérente d'environ 0,16-0,24 dl/g (**R202H**).

2. Implant biodégradable selon la revendication 1, dans lequel l'implant ne contient pas de prostamide.

3. Implant biodégradable selon la revendication 1, dans lequel ledit filament extrudé comprend environ 5 à environ 10 % en poids de PEG 3350.

4. Implant biodégradable selon la revendication 1, ledit implant étant sous la forme d'un filament extrudé et consistant en environ 20% en poids de latanoprost, environ 40% en poids d'un poly(D,L-lactide) à terminaison ester ayant une viscosité inhérente d'environ 0,16-0,24 dl/g (**RG202S**) ; et environ 40% en poids d'un poly(D,L-lactide) à terminaison acide ayant une viscosité inhérente d'environ 0,16-0,24 dl/g (**R202H**).

5. Procédé de fabrication d'un implant intraoculaire biodégradable selon la revendication 1, le procédé comprenant les étapes consistant à :
a) mélanger le latanoprost, le (les) polymère(s) biodégradable(s) et optionnellement le PEG pour former un mélange ; et
b) extruder le mélange à une température inférieure à environ 80°C pour former un filament qui va libérer le latanoprost de façon continue pendant au moins 30 jours après la mise en place dans l'oeil de l'individu.
